# EUROPEAN PATENT APPLICATION

(11) **EP 4 759 806 A1**
(43) Date of publication of application: **17.06.2026**
(21) Application number: 24219252.4
(22) Date of filing: 11.12.2024
(51) Int. Cl.: C07D 319/12

(54) **A PROCESS FOR RECYCLING POLYHYDROXY ACID**

(71) Applicant: Sulzer Management AG, 8401 Winterthur (CH)
(72) Inventor: The designation of the inventor has not yet been filed

(57) **Abstract**

The present invention relates to a process of producing a cyclic ester of a hydroxy acid comprising the steps of:
a) providing a crude composition containing oligomer of the hydroxy acid, wherein the step of providing the crude composition comprises subjecting a polymer composition containing polymer of the hydroxy acid in the presence of a i) polyalcohol comprising at least three hydroxy groups and/or ii) a primary amine comprising at least one amino group to a depolymerization reaction so as to obtain a depolymerized composition comprising oligomer of the hydroxy acid, and
b) subjecting the crude composition obtained in step a) to a cyclodepolymerization reaction so as to obtain a composition containing cyclic ester of the hydroxy acid.

## Description

The present invention relates to a process of producing a cyclic ester of a hydroxy acid, in particular an w-hydroxy acid (omega-hydroxy acid) or an α-hydroxy acid (alpha-hydroxy acid), such as lactide, from polyhydroxy acid, in particular poly-ω-hydroxy acid or poly-α-hydroxy acid, such as from polylactic acid, for example from waste polylactic acid, and thus for recycling the polyhydroxy acid.

Polyhydroxy acid homo- or copolymers are of particular interest, because they can be obtained from renewable resources and are mostly compostable and/or biodegradable. Moreover, the technological properties of these polymers come quite close to the properties of those polymers derived from fossil-based resources, which explains why these polymers are regarded as highly promising substitutes for the latter. One example for a commercially important polyhydroxy acid homopolymer is polylactic acid, which is based on an α-hydroxy acid, namely on lactic acid, and which has a wide range of applications. For instance, polylactic acid is used in the biomedical field in chirurgical implants, in films, such as e.g. in packaging, in fibers, such as e.g. for garments, in hygienic articles, in carpets and in disposable plastic products, such as e.g. disposable cutlery or containers. Moreover, polylactic acid has found wide application in composite materials, such as in fiber-reinforced plastics. Another important example for a respective polyhydroxy acid homopolymer is polycaprolactone, which is a poly-w-hydroxy acid and is derived from a cyclic ester, caprolactone, originated from the intramolecular esterification of 6-hydroxyhexanoic acid. This polymer finds widespread applications in the production of speciality polyurethanes and is characterized by a good resistance to water, to oil and to solvent. Other examples are polyglycolic acid, polyvalerolac-tone and the like. Polyhydroxy acid copolymers are interesting alternatives, since by an appropriate selection of the comonomers and their relative amounts to each other and by adjusting an appropriate molecular weight, certain properties of the copolymers may be tailored to the intended use.

Generally, two alternative principal methods are known for synthesizing polyhydroxy acid homo- or copolymers. The first principal method is the direct polycondensation of one or more aliphatic hydroxy acid(s) to the respective homopolymer or copolymer, respectively, such as the direct polycondensation of lactic acid to polylactic acid. However, this principal method only leads to low molecular weight (co)polymers and is thus limited to specific (co)polymers. The second principal method is the ring-opening-polymerization of one or more cyclic esters of hydroxy acid(s), such as the ring-opening-polymerization of lactide (which is the cyclic diester of lactic acid), glycolide (which is the cyclic diester of glycolic acid), lactones (which are cyclic monoesters) or the like. This is the preferred method nowadays for the industrial production of polylactic acid and other polyhydroxy acid homo- or copolymers. The cyclic esters may be produced by intramolecular esterification of an aliphatic hydroxy acid, such as in the case of a lactone, for instance caprolactone, or by condensation of two hydroxy acid molecules into a cyclic diester, such as the condensation of lactic acid to lactide or the condensation of glycolic acid to glycolide. Alternatively, the cyclic ester, such as in particular cyclic diester, may be produced by first oligomerizing a hydroxy acid and then subjecting the oligomer to a depolymerization reaction in order to obtain the cyclic diester. For instance, lactide is often prepared by the latter method, for instance by fermentation of carbohydrates from biomass, such as starch, sugar or corn resulting in lactic acid, by then oligomerizing the lactic acid and by afterwards subjecting the oligomers to a depolymerization reaction in order to obtain lactide. After purification, the one or more cyclic esters of hydroxy acid as monomer(s) are then polymerized in the presence of a catalyst and optionally an initiator to form high molecular weight polymer.

In view of the increasing amounts of articles made of polyhydroxy acid, the recycling of polyhydroxy acid waste becomes more and more important. In order to recycle polylactic acid waste, processes for converting polylactic acid to lactide, before the lactide is used as starting material for preparing new polylactic acid, have been already proposed. In these processes, the polylactic acid waste is first depolymerized to lactic acid oligomer, before the lactic acid oligomer is cyclodepolymerized to lactide. However, the known recycle processes have several drawbacks, such as that they require in both steps of depolymerizing polyhydroxy acid to lactic acid oligomer and of cyclodepolymerizing lactic acid oligomer to lactide a comparable long reaction time at an elevated temperature so that the respective processes are quite energy consuming. Moreover, these processes often lead to an undesired racemization of the lactide, which is chiral and exists in the form of enantiomeric L-lactide, D-lactide and meso-lactide. Analogously, lactic acid is chiral and exists in the form of enantiomeric L-lactic acid and D-lactic acid. Since the properties of the polylactic acid, which is produced from the lactide, such as the processing properties, crystallization properties and degradation behavior of polylactic acid, depend on the structure and composition of the polymer chains, in particular on the ratio of the L- to the D-stereoisomer of lactic acid, processes are desirable, in which lactide with a desired ratio of the three enantiomers is obtained.

In view of this, the object underlying the present invention is to provide a process of converting directly polyhydroxy acid, in particular w-hydroxy acid or α-hydroxy acid, such as polylactic acid, in particular waste polylactic acid, to a cyclic ester of the hydroxy acid, such as lactide, wherein the process is fast, may be performed within a comparable short reaction time, is energy efficient and allows to obtain the cyclic ester of the hydroxy acid in the desired stereochemical form.

In accordance with the present invention this object is satisfied by providing a process of producing a cyclic ester of a hydroxy acid comprising the steps of:
a) providing a crude composition containing oligomer of the hydroxy acid, wherein the step of providing the crude composition comprises subjecting a polymer composition containing polymer of the hydroxy acid in the presence of a i) polyalcohol comprising at least three hydroxy groups and/or ii) a primary amine comprising at least one amino group to a depolymerization reaction so as to obtain a depolymerized composition comprising oligomer of the hydroxy acid, and
b) subjecting the crude composition obtained in step a) to a cyclodepolymerization reaction so as to obtain a composition containing cyclic ester of the hydroxy acid.

This solution bases on the surprising finding that by performing the depolymerization reaction of the polymer of the hydroxy acid or polyhydroxy acid, respectively, to oligomer of the hydroxy acid in the presence of the aforementioned depolymerizing agent as well as by performing the subsequent cyclodepolymerization reaction of the oligomer of the hydroxy acid to the cyclic ester of the hydroxy acid in the presence of polyalcohol and/or primary amine, both, the depolymerization reaction as well as the cyclodepolymerization reaction are enhanced. The aforementioned depolymerizing agent is i) a polyalcohol comprising at least three hydroxy groups and/or ii) a primary amine comprising at least one amino group. This allows to reduce the reaction times in both steps, whereby a racemization of the produced cyclic ester of the hydroxy acid is reliably avoided. Thereby, the process is energy efficient and thus characterized by low operational costs. Furthermore, the process is characterized by a high yield, since side-reactions during both steps a) and b) are minimized. All in all, the process in accordance with the present invention is fast and may thus be performed within a comparable low reaction time, is energy efficient and allows to obtain the cyclic ester of the hydroxy acid in the desired stereochemical form.

The term polymer as used herein means in accordance with the present invention any kind of polymer and thus covers homopolymers as well copolymers.

In accordance with the present invention, polymers are macromolecules being composed of at least 60 repeating subunits, whereas oligomers are macromolecules being composed of 2 to 59 repeating subunits.

In turn, cyclic ester means in accordance with the present invention any cyclic molecule containing an ester group in the cyclic structure. In other words, a cyclic molecule containing an ester group only as attached side group, but not in the cyclic structure, is not considered in the present invention as cyclic ester. The cyclic ester may be a cyclic monoester, such as in the case of lactones, for instance ε-caprolactone or γ-valerolactone, or a cyclic diester, such as in the case of lactide and glycolide.

In accordance with the present invention, in the first step a) polymer of the hydroxy acid is depolymerized to an oligomer of the hydroxy acid. The polymer of the hydroxy acid is preferably waste polymer of the hydroxy acid so that in fact the process is a recycling process. The waste polymer may be pre-consumer waste, a post-consumer waste or a mixture of both.

As set out above, the depolymerization reaction as well as the cyclodepolymerization reaction are performed in the presence of a depolymerizing agent, wherein the depolymerizing agent is i) a polyalcohol comprising at least three hydroxy groups and/or ii) a primary amine comprising at least one amino group. The polyalcohol and/or primary amine is/are not consumed during the depolymerization reaction of the polymer of the hydroxy acid to the oligomer of the hydroxy acid and is/are thus still contained in the depolymerized composition and consequently in the crude composition, which is subjected in step b) to the cyclodepolymerization reaction.

Good results are in particular obtained, when in step a) a trivalent and/or tetravalent polyalcohol and in particular a polyalcohol is used, which is selected from the group consisting of glycerol, phloroglucinol, pentaerythritol, sorbitol, dipentaerythritol, tripentaerythritol, 1,1,1-tris (hydroxymethyl)ethane, 1,1,1-tris(hydroxymethyl)propane, di-(trimethylolpropane), trimethylolpropanethoxylate, polyphenols comprising at least three hydroxy groups, saccharides comprising at least three hydroxy groups, star-shaped oligomers comprising at least three hydroxy end groups, cyclitol and arbitrary combinations of two or more of the aforementioned polyalcohols.

Also, good results are in particular obtained, when in step a) a primary amine comprising at least one amino group and preferably a primary amine comprising at least two amino groups is used. The primary amine comprising at least one amino group may or may not comprise any further functional groups in addition to the at least one amino group. For instance, the primary amine comprising at least one amino group may comprise one or more hydroxyl groups, i.e. may be an aminoalcohol, such as one according to the formula OH-R-NH₂, wherein R is a hydrocarbon group, such as an alkylene group, such as a C₁₋₅-alkylene group. Preferably, the primary amine comprising at least one amino group is an aminoalcohol or a primary amine comprising no other functional group in addition to the at least one amino group. Suitable examples for primary amines comprising at least one amino group are those being selected from the group consisting of ethylenediamine, hexamethylenediamine, tris(2-aminoethyl)amine, 1-aminopropan-2-ol, 5-amino-1-pentanol, 3-amino-1-propanol, 3-amino-1,2-propanediol and arbitrary combinations of two or more of the aforementioned primary amines.

Preferably, the depolymerization reaction in step a) is performed with a polymer composition comprising, based on 100% by mol of the reaction composition, 50 to 100% by mol and preferably 90 to 99.9% by mol of the polymer of the hydroxy acid and 0 to 50% by mol and preferably 0.1 to 10% by mol of the polyalcohol and/or the primary amine.

In accordance with a particular preferred embodiment of the present invention, the reaction composition further comprises - in addition to the polymer of the hydroxy acid and in addition to the polyalcohol and/or the primary amine - water and/or the hydroxy acid. "The hydroxy acid" means here and below that hydroxy acid, which constitutes the polymer of the hydroxy acid being contained in the polymer composition. More preferably, the reaction composition further comprises water and the hydroxy acid. It has been found during the present invention that by performing the depolymerization reaction in the presence of the i) polyalcohol comprising at least three hydroxy groups and/or ii) the primary amine comprising at least one amino group and in the presence of water and/or the hydroxy acid and in particular by performing the depolymerization reaction in the presence of the i) polyalcohol comprising at least three hydroxy groups and/or ii) the primary amine comprising at least one amino group, in the presence of water as well as in the presence of the hydroxy acid, the suppression of the formation of side-products and thus the increase of yield as well as the suppression of racemization are assisted.

Good results are in particular obtained, when the reaction composition further comprises - in addition to the polymer of the hydroxy acid and in addition to the polyalcohol and/or the primary amine -, based on 100% by mol of the reaction composition, 0 to 20% by mol and more preferably 0.1 to 10% by mol of water and/or 0 to 10% by mol and more preferably 0.1 to 10% by mol of the hydroxy acid.

In a further development of the idea of the present invention, it is proposed that the reaction composition further comprises a catalyst. Thereby, possible side-reactions may be minimized and the yield may be increased. Moreover, the presence of catalyst assists in the shortening of the required reaction time and of the required reaction temperature. Examples for suitable catalysts are metal oxides, metal carbonates, metal bicarbonates and organometallic compounds. Suitable examples for metal oxides are transition metal oxides, and suitable examples for organometallic compounds are those comprising a metal selected from the group consisting of magnesium, titanium, zinc, aluminum, indium, yttrium, tin, lead, antimony, bismuth and any combination of two or more of the aforementioned metals and an organic residue being selected from the group consisting of alkyl groups, aryl groups, halides, oxides, alkanoates, alkoxides and any combination of two or more of the aforementioned groups. Suitable examples for metal carbonates and metal bicarbonates are alkali metal carbonates, alkaline earth metal carbonates, alkali metal bicarbonates and alkaline earth metal bicarbonates. Particular suitable examples for catalysts are catalysts being selected from the group consisting of tin oxide, iron oxide, copper oxide, tin octanoate, butyl tin oxide, calcium carbonate, potassium carbonate and arbitrary combinations of two or more of the aforementioned catalysts.

Preferably, the polymer composition contains, based on 100% by mol of the polymer composition, 0 to 5,000 ppm and more preferably 100 to 1,000 ppm of catalyst.

As set out above, the present invention is not particularly limited concerning the kind of used polymer of the hydroxy acid and of produced cyclic ester of hydroxy acid, as long as the kind of hydroxy acid of the polymer and of the cyclic ester is the same. Preferably, the hydroxy acid is a w-hydroxy acid or α-hydroxy acid and hence the polymer is a poly-w-hydroxy acid or a poly-α-hydroxy acid. The present invention is particularly suitable for using a polymer of a w-hydroxy acid or a α-hydroxy acid being selected from the group consisting of lactic acid, glycolic acid, 6-hydroxyhexanoic acid, 5-hydroxypentanoic acid and arbitrary combinations of two or more of the aforementioned acids and thus for producing a cyclic ester being selected from the group consisting of lactide, glycolide, caprolactone, valerolactone and arbitrary combinations of two or more of the aforementioned cyclic esters. Hence, preferred hydroxy acids are those being selected from the group consisting of lactic acid, glycolic acid, 6-hydroxyhexanoic acid, 5-hydroxypentanoic acid and arbitrary combinations of two or more of the aforementioned acids.

Optionally, the polymer composition may further contain an oligomer of the hydroxy acid having a weight average molecular weight of 100 to 500 g/mol, such as 0 to 20% by mol and more preferably 0.1 to 10% by mol, of oligomer.

As set out above, the presence of the i) polyalcohol comprising at least three hydroxy groups and/or ii) the primary amine comprising at least one amino group and of the optional water, hydroxy acid and catalyst allows to reduce the reaction time and the reaction temperature so as to reduce the operational costs. Good results are in particular obtained, when the depolymerization reaction of step a) is performed at a temperature of 120 to 220°C, more preferably 160 to 200°C and most preferably 170 to less than 190°C. A reaction temperature of less than 190°C is particularly preferred for depolymerizing polylactic acid, since thereby a racemization of the enantiomers is reliably avoided.

Concerning the pressure, the depolymerization reaction is not particularly restricted. Good results are in particular obtained, when the depolymerization reaction of step a) is performed at ambient pressure or at an over atmospheric pressure of up to 3 MPa.

Advantageously, the depolymerization reaction is performed for a comparable short reaction time of 1 hour or less, such as performed for a reaction time between 15 and less than 60 minutes.

The depolymerization reaction may be performed in any kind of reactor, such as in a batch reactor, in a continuous reactor or in a reactive extrusion reactor, preferably in an extruder or plug-flow reactor.

In a further development of the idea of the present invention, it is proposed that the oligomer of the hydroxy acid contained in the depolymerized composition obtained in the depolymerization reaction of step a) has a weight average molecular weight of 150 to 5,000 g/mol and more preferably of 200 to 1,000 g/mol. In accordance with the present invention, the number- and weight-average molecular weight (Mₙ and M_{w}) of polymers is determined by gel permeation chromatography using a poly(methyl methacrylate) standard and a sample concentration of 1 to 5 mg/ml in 1ml HFIP depending on the sample's molecular weight, wherein the column temperature is 40 °C, the temperature of the RI-Detector (refractive index) is 40 °C and the flow rate 1 ml/min. As instrument, (GPC Viscotek TDA max from Malvern Panalytical, UK equipped with a Viscotek VE 2001 solvent/sample module, a pre-column HFIP guard (50 mm length and 8 mm internal diameter), two columns (Viscotek HFIP6000M and HFIP3000, Viscotek, Switzerland; 300 mm length and 8 mm internal diameter), and a triple detector Viscotek TDA 305 (RI, UV and viscosimeter) may be used. The calibration curve may be constructed using poly(methylmethacrylate) (PMMA) standard (Mn,max = 50,352 g/mol and Ð of 1.023).

In accordance with a further particular preferred embodiment of the present invention, the depolymerized composition comprising oligomer of the hydroxy acid obtained in the depolymerization reaction of step a) is used as crude composition in step b).

Alternatively, it is possible to combine the aforementioned step of subjecting the polymer composition to a depolymerization reaction with one or more other reactions leading to oligomer of the hydroxy acid, for instance by mixing the depolymerized composition obtained in the depolymerization reaction of step a) with one or more compositions and in particular with one or more compositions containing oligomer of the hydroxy acid derived from other reation(s) so as to obtain the crude composition, which is subjected in step b) to the cyclodepolymerization step.

For instance, the step a) of the process in accordance with the present invention further comprises in the aforementioned embodiment a sub-step of reacting a starting composition containing the hydroxy acid, such as lactic acid, to a reaction composition containing oligomer of the hydroxy acid, wherein at least a portion of the reaction composition is mixed with at least a portion of the depolymerized composition obtained in the aforementioned depolymerization reaction so as to obtain the crude composition, which is subjected in step b) to the cyclodepolymerization step. Particularly preferably, all the reaction composition is mixed with all of the depolymerized composition obtained in the aforementioned depolymerization reaction so as to obtain the crude composition, which is subjected in step b) to the cyclodepolymerization step. The oligomer obtained by reacting the starting composition containing the hydroxy acid has preferably a weight average molecular weight of 150 to 5,000 g/mol and more preferably of 200 to 1,000 g/mol.

Furthermore, it is preferred that the starting composition is obtained by dewatering a mixture of water and lactic acid, such as a respective aqueous composition having been obtained by fermentation of carbohydrates from biomass, such as starch, sugar or corn.

In a further development of the idea of the present invention, it is suggested that the sub-step of reacting a starting composition containing the hydroxy acid to a reaction composition containing oligomer of the hydroxy acid is performed in two or more subsequent reactors. Preferably, the starting composition is fed into the first of the two or more subsequent reactors and reacted therein to a first reaction composition, wherein the first reaction composition is removed from the first reactor. The removed first reaction composition is then fed into the second reactor and reacted therein to a second reaction composition, wherein the second reaction composition is removed from the second reactor. If more than two reactors are used, the removed second reaction composition is fed into the next reactor and reacted therein to a next reaction composition, which is removed from the respective reactor and which is, if one or more further reactors are used, led through all further reactors so as to obtain a final reaction composition. At least a portion of the depolymerized composition obtained in the depolymerization reaction is added to the final reaction composition so as to obtain the crude composition being used in step b). Optionally, one or more other portions of the depolymerized composition is/are added to one or more of the reaction compositions being removed from the first to the penultimate reactor, before the so obtained mixture is fed into the next reactor.

Preferably, the sub-step of reacting the starting composition containing the hydroxy acid to a reaction composition containing oligomer of the hydroxy acid is performed in two to ten, more preferably in two to five, yet more preferably in two to four, still more preferably in two or three and most preferably in three subsequent reactors.

In order to shift the equilibrium of the reaction to the product, it is proposed in a further development of the idea of the present invention to remove separately from the reaction mixture, for instance by evaporation, water obtained as reaction by-product from any of the reactors, in which the sub-step of reacting the starting composition containing the hydroxy acid to a reaction composition containing oligomer of the hydroxy acid is performed.

In a preferred variant of the aforementioned embodiment, the sub-step of reacting the starting composition containing the hydroxy acid to a reaction composition containing oligomer of the hydroxy acid is performed in three subsequent reactors.

The starting composition is fed into the first of the three subsequent reactors and reacted therein to a first reaction composition, wherein the first reaction composition is removed from the first reactor and is fed into the second reactor and reacted therein to a second reaction composition, wherein the second reaction composition is removed from the second reactor and is fed into the third reactor and reacted therein to a third reaction composition, wherein the third reaction composition is removed from the third reactor. Each a portion of the depolymerized composition obtained in the depolymerization reaction is added to any of the three reaction compositions, wherein the composition being obtained by mixing the third reaction composition with a portion of the depolymerized composition is used as crude composition in step b).

Again, in order to shift the equilibrium of the reaction to the product, it is suggested in a further development of the idea of the present invention to remove separately from the reaction mixture, for instance by evaporation, water obtained as reaction by-product from any of the reactors, in which the sub-step of reacting the starting composition containing the hydroxy acid to a reaction composition containing oligomer of the hydroxy acid is performed.

In a further particular preferred variant of the aforementioned embodiment of the present invention, the starting composition further contains alcohol and preferably polyalcohol comprising at least three hydroxy groups, wherein a portion of each of the reaction mixtures being removed from the reactor(s) is recycled into the polymer composition used in step a), whereas the remaining portion of each of the reaction mixtures being removed from the reactor(s) is fed into the next reactor. For instance, the content of alcohol in the starting composition is, based on 100% by mol of the starting composition, 0 to 20% by mol and more preferably 0.1 to 10% by mol.

Concerning the reaction conditions of the cyclodepolymerization reaction of step b) the present invention is not particularly limited. Good results are in particular obtained, when the cyclodepolymerization reaction of step b) is performed at a temperature of 150 to 230°C. More preferably, the cyclodepolymerization reaction of step b) is performed at a temperature of 170 to 210°C and most preferably 170 to less than 200°C. A reaction temperature of less than 190°C is particularly preferred for cyclodepolymerizing lactic acid oligomer to lactide, since thereby a racemization of the enantiomers is reliably avoided.

In addition, it is preferred that the cyclodepolymerization reaction of step b) is performed at ambient pressure or at subatmospheric pressure. If the cyclodepolymerization reaction of step b) is performed at subatmospheric pressure, the pressure is preferably 0.1 to 10 kPa and most preferably 0.1 to 1 kPa.

Advantageously, the cyclodepolymerization reaction is performed for a short reaction time of 30 to 240 minutes and more preferably of 60 to 120 minutes.

The cyclodepolymerization reaction may be performed in any kind of reactor, such as in a batch reactor, in a continuous reactor or in a reactive extrusion reactor, preferably in an extruder or plug-flow reactor.

In a further development of the idea of the present invention, it is proposed that the cyclodepolymerization reaction is performed in the presence of a catalyst, i.e. that the crude composition used in step b) further comprises a catalyst. Thereby, possible side-reactions may be minimized and the yield increased. Moreover, the presence of catalyst assists in the shortening of the required reaction time and of the required reaction temperature. As catalyst for the cyclodepolymerization reaction the same catalyst as described further above for the depolymerization reaction may be used, i.e. examples for suitable catalysts are metal oxides, metal carbonates, metal bicarbonates and organometallic compounds. Suitable examples for metal oxides are transition metal oxides, and suitable examples for organometallic compounds are those comprising a metal selected from the group consisting of magnesium, titanium, zinc, aluminum, indium, yttrium, tin, lead, antimony, bismuth and any combination of two or more of the aforementioned metals and an organic residue being selected from the group consisting of alkyl groups, aryl groups, halides, oxides, alkanoates, alkoxides and any combination of two or more of the aforementioned groups. Suitable examples for metal carbonates and metal bicarbonates are alkali metal carbonates, alkaline earth metal carbonates, alkali metal bicarbonates and alkaline earth metal bicarbonates. Particular suitable examples for catalysts are catalysts being selected from the group consisting of tin oxide, iron oxide, copper oxide, tin octanoate, butyl tin oxide, calcium carbonate, potassium carbonate and arbitrary combinations of two or more of the aforementioned catalysts.

Preferably, the crude composition used in step b) contains, based on 100% by mol of the polymer composition, 0 to 5,000 ppm and more preferably 100 to 1,000 ppm of catalyst.

In accordance with another preferred embodiment of the present invention, a portion of the composition containing cyclic ester of the hydroxy acid obtained in step b) is recycled into the polymer composition used in step a).

In order to increase the purity of the cyclic ester of the hydroxy acid obtained in step b), it is proposed in a further development of the idea of the present invention that the composition containing the cyclic ester of the hydroxy acid obtained in step b) is purified in a subsequent step c). Preferably, the composition containing the cyclic ester of the hydroxy acid obtained in step b) is purified in a subsequent step c) by a method being selected from the group consisting of distillation, static crystallization, falling film crystallization or suspension crystallization.

For instance, the purification of step c) comprises at least one static crystallization step, wherein the at least one static crystallization step preferably comprises one to four static crystallization stages and more preferably two static crystallization stages.

In accordance with an alternative variant, the purification of step c) comprises at least one dynamic crystallization step, wherein the at least one dynamic crystallization step preferably comprises one to four dynamic crystallization stages. More preferably, the dynamic crystallization is a falling film crystallization.

In accordance with an alternative variant, the purification of step c) comprises at least one distillation step each of which being performed in a distillation column. More preferably, the hydroxy acid is lactic acid and step c) comprises two distillation steps each of which being performed in a distillation column, wherein in the first distillation column the composition containing lactide as the cyclic ester of lactic acid obtained in step b) is separated into an overhead fraction, into a bottom fraction and into a side fraction, wherein the side fraction of the first distillation column is fed as side fraction into the second distillation column, in which a meso-lactide enriched composition is produced as overhead fraction and a L-lactide enriched composition is produced as side fraction or as bottom fraction.

Subsequently, the present invention is described by means of illustrative, but not limiting figures, in which:
- Fig. 1: shows a scheme of a process of producing lactide in accordance with one embodiment of the present invention.
- Fig. 2: shows a scheme of a process of producing lactide in accordance with another embodiment of the present invention.
- Fig. 3: shows a scheme of a process of producing lactide in accordance with another embodiment of the present invention.

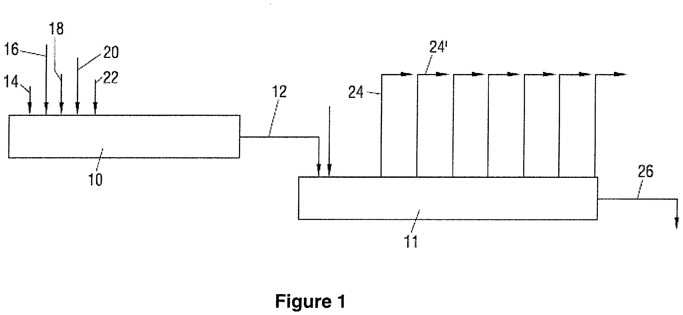

The process being schematically shown in figure 1 is performed in two subsequent plug-flow reactors 10, 11, which are connected with each other by a line 12. The first plug-flow reactor 10 comprises an inlet line 14 for polylactic acid, an inlet line 16 for catalyst, an inlet line 18 for lactic acid, an inlet line 20 for i) polyalcohol comprising at least three hydroxy groups and/or ii) a primary amine comprising at least one amino group as well as an inlet line 22 for water, whereas the second plug-flow reactor 11 comprises seven subsequent outlet lines 24, 24' for lactide and an outlet line 26 for residue. The polylactic acid, the lactic acid, the i) polyalcohol comprising at least three hydroxy groups and/or ii) the primary amine comprising at least one amino group, the water and the catalyst are mixed in the first plug-flow reactor 10 to a polymer composition, which is reacted within the first plug-flow reactor 10 in a depolymerization reaction to a depolymerized composition comprising lactic acid oligomer. The depolymerized composition is then led via line 12 into the second plug-flow reactor 11, in which it is subjected to a cyclodepolymerization reaction so as to obtain a composition containing lactide. The lactide is successively withdrawn from the second plug-flow reactor 11 via the outlet lines 24, 24', whereas the residue is withdrawn from the second plug-flow reactor 11 via the outlet line 26.

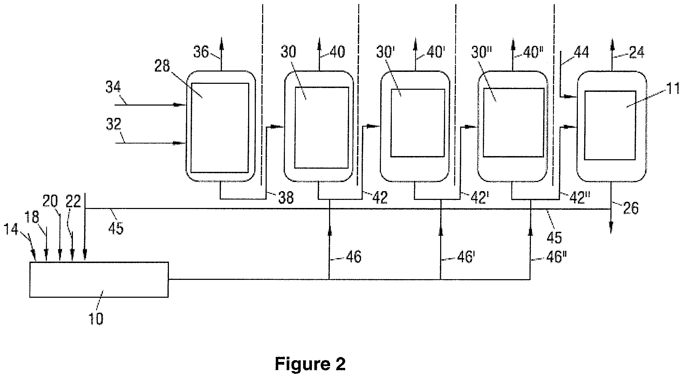

The process being schematically shown in figure 2 differs from that being shown in figure 1 in that the step of subjecting the polylactic acid to a depolymerization reaction is combined with another step of reacting lactic acid to lactic acid oligomer, before both oligomers are subjected to the cyclodepolymerization step. More specifically, the plant, in which the process shown in figure 2 is performed, comprises in addition to the first plug-flow reactor 10 and to a second reactor 11, a dewatering vessel 28 and three subsequent prepolymerization reactors 30, 30', 30". While the dewatering vessel 28 comprises an inlet line 32 for water, an inlet line 34 for lactic acid, an outlet line 36 for water and an outlet line 38 for a dewatered composition, each of the prepolymerization reactors 30, 30', 30" comprises an outlet line 40, 40', 40" for water as well as an outlet line 42, 42', 42" for an oligomer containing composition, wherein the last outlet line 42" for oligomer containing composition is in fact an outlet line for the crude composition. The second reactor 11 is connected with the outlet line 42" for oligomer containing composition or crude composition, respectively, as well as with an inlet line 44 for catalyst, with the outlet line 24 for lactide and with the outlet line 26 for residue. Moreover, a recycle line 45 leads form the second reactor 11 to the first reactor 10 and from the first reactor 10 three outlet lines 46, 46', 46" lead each to one of the outlet lines 42, 42', 42". During the operation, lactic acid oligomer is produced in the first reactor 10 from the polylactic acid by a depolymerization reaction, whereas dewatered composition comprising lactic acid and water is reacted in the first prepolymerization reactor 30 to lactic acid oligomer. The produced lactic acid oligomer being produced in the first prepolymerization reactor 30 together with a portion of the lactic acid oligomer being produced in the first reactor 10 and being added to the outlet line 42 via outlet line 46 is then further reacted in the second prepolymerization reactor 30', whereas the produced lactic acid oligomer being produced in the second prepolymerization reactor 30' together with a portion of the lactic acid oligomer being produced in the first reactor 10 and being added to the outlet line 42' via outlet line 46' is then further reacted in the third prepolymerization reactor 30". The oligomer containing composition or crude composition, respectively, which is withdrawn from the third prepolymerization reactor 30" is led together with a portion of the lactic acid oligomer being produced in the first reactor 10 and being added to the outlet line 42" via outlet line 46" via line 46" into the second reactor 11, in which it is cyclodepolymerized into lactide. Remaining oligomer is partially withdrawn from the second reactor 11 via the line 26, whereas the remaining portion thereof is recycled via the recycle line 45 into the first reactor 10. Water is withdrawn from each of the dewatering vessel 28 and of the prepolymerization reactors 30, 30', 30" via the lines 36, 40, 40', 40".

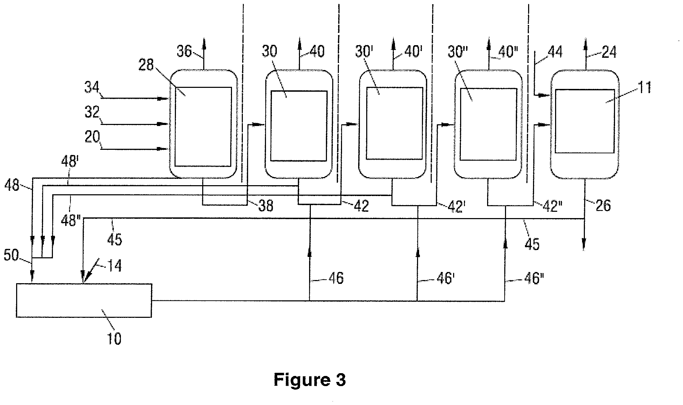

The process being schematically shown in figure 3 differs from that being shown in figure 2 in that the inlet line 20 for i) polyalcohol comprising at least three hydroxy groups and/or ii) primary amine comprising at least one amino group leads into the dewatering vessel 28, that the first reactor does not comprise inlet lines 18, 20, 22 for lactic acid, i) polyalcohol comprising at least three hydroxy groups and/or ii) primary amine comprising at least one amino group and water, but recycle lines 48, 48', 48"leading from each of the dewatering vessel 28, the first prepolymerization reactor 30 and the second prepolymerization reactor 30' via line 50 into the first reactor.

Subsequently, the present invention is described by means of illustrative, but not limiting examples.

### Comparative Example 1

A chain scission reaction of polylactic acid waste in the presence of ethylene alcohol was performed by adding polylactic acid waste and 2% by weight of ethylene glycol into a reactor and heating, stirring and reacting the mixture in the reactor at a temperature of 200°C for a reaction time of 120 minutes so as to prepare polylactic acid oligomer having a weight average molecular weight of 2,592 g/mol.

Thereafter, 0.3% by weight of a cracking catalyst, namely Sn(Oct)₂, have been added to the prepared polylactic acid oligomer and mixed, before the so obtained mixture was subjected to a reduced pressure distillation operation at a vacuum of 0.5 kPa and to a continuously cracking of the lactic acid oligomer at a temperature of 195°C, before the so obtained lactide was collected after 180 minutes.

The collected crude lactide has a purity of 93%.

### Example 1

A chain scission reaction of polylactic acid waste has been performed in the presence of 2% by weight of trimethylolpropane ethoxylate having a number average molecular weight of 170 g/mol under stirring and constantly heating at 180°C for 40 minutes. The obtained oligomer has a wight average molecular weight of 14,000 g/mol.

The addition of 0.4% by weight of Sn(Oct)₂ for further depolymerization yielded 7.8 kg/mol oligomers.

Thereafter, the oligomer mixture has been heated to 195°C at a vacuum of 5 to 6 mbar for 70 minutes, which yielded 95% of crude lactide having a purity of 97%.

Compared with the comparative example 1, example 1 was characterized by a higher reactivity, i.e. lower reaction time, by a higher purity of the collected lactide and by less racemization.

### Example 2:

A chain scission reaction of polylactic acid waste has been performed in the presence of 1% by weight of dipentaerythritol under stirring and constantly heating at 195°C for 60 minutes. The obtained oligomer has a wight average molecular weight of 30,000 g/mol.

Thereafter, the oligomer mixture has been heated to 190°C at a vacuum of 5 to 6 mbar for 60 minutes, which yielded 92% of crude lactide having a purity of 98%.

### Example 3:

A chain scission reaction of polylactic acid waste has been performed in the presence of 5% by weight of tripentaerythritol under stirring and constantly heating at 200°C for 60 minutes. The obtained oligomer has a wight average molecular weight of 15,000 g/mol.

Thereafter, 0.2% by weight of Sn(Oct)₂ were added as catalyst and the so obtained oligomer mixture was heated to 190°C at a vacuum of 5 to 6 mbar for 60 minutes, which yielded 96% of crude lactide having a purity of 93%.

### Example 4:

A chain scission reaction of a mixture of 50 g polylactic acid waste and 50 g polycprolactone waste has been performed in the presence of 12% by weight of trimethylolpropane ethoxylate having a number average molecular weight of 170 g/mol under stirring and constantly heating at 160°C for 60 minutes. The obtained oligomer has a wight average molecular weight of 20,000 g/mol.

Thereafter, 0.3% by weight of Sn(Oct)₂ were added as catalyst and the so obtained oligomer mixture was heated to 195°C at a vacuum of 5 to 6 mbar for 90 minutes, which yielded 95% of crude lactide having a purity of 90%.

### Reference numerals

- 10: First reactor
- 11: Second reactor
- 12: Line
- 14: Inlet line for polylactic acid
- 16: Inlet line for catalyst
- 18: Inlet line for lactic acid
- 20: Inlet line for polyalcohol and/or primary amine
- 22: Inlet line for water
- 24, 24': Outlet lines of second reactor
- 26: Outlet line for residue
- 28: Dewatering vessel
- 30, 30', 30": Prepolymerization reactor
- 32: Inlet line for water
- 34: Inlet line for lactic acid
- 36: Outlet line for water
- 38: Outlet line for dewatered composition
- 40, 40', 40": Outlet line for water
- 42, 42', 42": Outlet line for an oligomer containing composition
- 44: Inlet line for catalyst
- 45: Recycle line
- 46, 46', 46": Outlet line from first reactor
- 48, 48', 48": Recycle lines
- 50: Line

## Claims

1. A process of producing a cyclic ester of a hydroxy acid comprising the steps of:
a) providing a crude composition containing oligomer of the hydroxy acid, wherein the step of providing the crude composition comprises subjecting a polymer composition containing polymer of the hydroxy acid in the presence of i) a polyalcohol comprising at least three hydroxy groups and/or ii) a primary amine comprising at least one amino group to a depolymerization reaction so as to obtain a depolymerized composition comprising oligomer of the hydroxy acid, and
b) subjecting the crude composition obtained in step a) to a cyclodepolymerization reaction so as to obtain a composition containing cyclic ester of the hydroxy acid.

2. The process in accordance with claim 1, wherein in step a) a polyalcohol comprising at least three hydroxy groups is used, which is selected from the group consisting of glycerol, phloroglucinol, pentaerythritol, sorbitol, dipentaerythritol, tripentaerythritol, 1,1,1-tris (hydroxymethyl)ethane, 1,1,1-tris(hydroxymethyl)propane, di-(trimethylolpropane), trimethylolpropanethoxylate, polyphenols comprising at least three hydroxy groups, saccharides comprising at least three hydroxy groups, star-shaped oligomers comprising at least three hydroxy end groups, cyclitol and arbitrary combinations of two or more of the aforementioned polyalcohols.

3. The process in accordance with claim 1 or 2, wherein in step a) primary amine comprising at least one amino group is used, which is selected from the group consisting of ethylenediamine, hexamethylenediamine, tris(2-aminoethyl)amine, 1-aminopropan-2-ol, 5-amino-1-pentanol, 3-amino-1-propanol, 3-amino-1 ,2-propanediol and arbitrary combinations of two or more of the aforementioned primary amines.

4. The process in accordance with any of the preceding claims, wherein the depolymerization reaction in step a) is performed with a polymer composition comprising, based on 100% by mol of the reaction composition, 50 to 100% by mol and preferably 90 to 99.9% by mol of the polymer of the hydroxy acid and 0 to 50% by mol and preferably 0.1 to 10% by mol of the i) polyalcohol comprising at least three hydroxy groups and/or ii) the primary amine comprising at least one amino group.

5. The process in accordance with claim 4, wherein the reaction composition further comprises water and/or the hydroxy acid, preferably, based on 100% by mol of the reaction composition, 0 to 20% by mol and more preferably 0.1 to 10% by mol of water and/or 0 to 10% by mol and more preferably 0.1 to 10% by mol of the hydroxy acid.

6. The process in accordance with any of the preceding claims, wherein the hydroxy acid is a w-hydroxy acid or a α-hydroxy acid and preferably selected from the group consisting of lactic acid, glycolic acid, 6-hydroxyhexanoic acid, 5-hydroxypentanoic acid and arbitrary combinations of two or more of the aforementioned acids.

7. The process in accordance with any of the preceding claims, wherein the depolymerization reaction of step a) is performed at a temperature of 120 to 220°C, preferably 160 to 200°C and more preferably 170 to less than 190°C.

8. The process in accordance with any of the preceding claims, wherein the oligomer of the hydroxy acid contained in the depolymerized composition obtained in the depolymerization reaction of step a) has a weight average molecular weight of 150 to 5,000 g/mol and preferably of 200 to 1,000 g/mol.

9. The process in accordance with any of the preceding claims, wherein the depolymerized composition comprising oligomer of the hydroxy acid obtained in the depolymerization reaction of step a) is used as crude composition in step b).

10. The process in accordance with any of claims 1 to 8, wherein the depolymerized composition comprising oligomer of the hydroxy acid obtained in the depolymerization reaction of step a) is mixed with one or more compositions containing oligomer of the hydroxy acid so as to obtain the crude composition being used in step b).

11. The process in accordance with claim 10, wherein step a) further comprises a sub-step of reacting a starting composition containing the hydroxy acid to a reaction composition containing oligomer of the hydroxy acid, wherein at least a portion and preferably all of the reaction composition is mixed with at least a portion and preferably all of the depolymerized composition obtained in step a) so as to obtain the crude composition being used in step b), wherein preferably the sub-step of reacting a starting composition containing the hydroxy acid to a reaction composition containing oligomer of the hydroxy acid is performed in two or more subsequent reactors, wherein the starting composition is fed into the first of the two or more subsequent reactors and reacted therein to a first reaction composition, wherein the first reaction composition is removed from the first reactor and is fed into the second reactor and reacted therein to a second reaction composition, wherein the second reaction composition is removed from the second reactor and, if more than two reactors are used, is fed into the next reactor and reacted therein to a next reaction composition, which is removed from the respective reactor and which is, if one or more further reactors are used, led through all further reactors so as to obtain a final reaction composition, wherein at least a portion of the depolymerized composition being obtained in step a) is added to the final reaction composition so as to obtain the crude composition being used in step b).

12. The process in accordance with claim 11, wherein a portion of the composition containing cyclic ester of the hydroxy acid obtained in step b) is recycled into the polymer composition used in step a).

13. The process in accordance with any of the preceding claims, wherein the cyclodepolymerization reaction of step b) is performed at a temperature of 150 to 230°C, preferably 170 to 210°C and more preferably 170 to less than 200°C, and wherein the cyclodepolymerization reaction of step b) is performed at ambient pressure or at a pressure of 0.1 to 10 kPa and preferably of 0.1 to 1 kPa.

14. The process in accordance with any of the preceding claims, wherein the composition containing the cyclic ester of the hydroxy acid obtained in step b) is purified in a step c) by a method being selected from the group consisting of distillation, static crystallization, falling film crystallization or suspension crystallization.

15. The process in accordance with claim 14, wherein the hydroxy acid is lactic acid, and wherein step c) comprises two distillation steps each of which being performed in a distillation column, wherein in the first distillation column the composition containing lactide as the cyclic ester of lactic acid obtained in step b) is separated into an overhead fraction, into a bottom fraction and into a side fraction, wherein the side fraction of the first distillation column is fed as side fraction into the second distillation column, in which a meso-lactide enriched composition is produced as overhead fraction and a L-lactide enriched composition is produced as side fraction or as bottom fraction.
